# EUROPEAN PATENT SPECIFICATION

(11) **EP 3 868 373 B1**
(45) Date of publication and mention of the grant of the patent: **30.08.2023**
(21) Application number: 18940322.3
(22) Date of filing: 12.11.2018
(51) Int. Cl.: A61K 31/353, A61K 31/352, A61K 31/05, A61P 17/00, A61P 17/04, A61P 25/00, A61K 9/08, A61K 9/20, A61K 9/48

(54) **USE OF A CANNABINOID COMPOSITION IN THE TREATMENT OF NEURODERMATITIS**
VERWENDUNG VON EINER CANNABINOID-VERBINDUNG BEI DER BEHANDLUNG VON NEURODERMITIS
UTILISATION D'UN COMPOSÉ CANNABINOÏDE POUR LE TRAITEMENT DE LA NEURODERMATITE

(43) Date of publication of application: 25.08.2021
(73) Proprietor: Hanyi Bio-Technology (Beijing) Co., Ltd., Beijing 100020 (CN)
(72) Inventor: ZHANG, Ke, Beijing 100020 (CN); TAN, Xin, Beijing 100020 (CN); CHANG, Tanran, Beijing 100020 (CN); JIN, Qian, Beijing 100020 (CN)
(74) Representative: Murgitroyd & Company
(86) International application number: PCT/CN2018/115075
(87) International publication number: WO 2020/097765

(56) References cited:
- EP-A1- 2 444 081
- WO-A1-2017/027553
- WO-A1-2017/055846
- WO-A1-2017/055846
- WO-A1-2018/071581
- WO-A1-2018/148785
- WO-A1-2018/148785
- CN-A- 1 547 479
- CN-A- 1 547 479
- CN-A- 109 200 046
- US-A1- 2012 295 968
- US-A1- 2012 295 968
- GALATHEA BISTERFELD VON MEER: "Juice from Cannabis Plants for Food/Beverage, Feed or Biogas", 9TH INTERNATIONAL CONFERENCE OF THE EUROPEAN INDUSTRIAL HEMP ASSOCIATION, 23 May 2012 (2012-05-23), pages 1-15, XP055032562,

## Description

### TECHNICAL FIELD

The present invention relates to the field of medicine, and in particular to a pharmaceutical composition comprising a cannabinoid combination or pharmaceutically available salts thereof for use in the treatment of neurodermatitis. The references to the methods of treatment by therapy in embodiment 13,14 and 15 of this description are to be interpreted as references to pharmaceutical compositions and medicaments of the present invention for use in those methods.

### BACKGROUND

Neurodermatitis, also known as lichen simplex chronicus, is a skin disease with limited cutaneous neurological dysfunction, and is a chronic skin disease characterized by paroxysmal pruritus and skin lichenification. The pathogenesis of the disease is still unclear, but it is generally believed that the disease is related to long-term scratching, friction, neuropsychiatric factors and certain external stimuli, among which, psychiatric factors are currently considered to be a major cause for the occurrence of the disease, and mood fluctuation, excessive mental tension, anxiety, sudden changes in living environment, etc. can make the disease worse and relapse. At present, the clinical treatment of neurodermatitis may be mostly performed by using histamines, calcium agents and the like to symptomatically relieve itching, with a supplement by oral administration of vitamin B. Sedatives may be selected for a patient with severe pruritus, and intravenous blocking of procaine or in combination with common threewingnut root drugs may be administrated for a patient with generalized rash. Although these treatments take effect quickly, they are only symptomatic treatment. Therefore, there is a disadvantage that the symptoms are treated but not the root cause, and the disease is prone to relapse.

Some studies have shown that neurodermatitis is significantly correlated with neuropsychiatric factors. In addition, long-term gastrointestinal dysfunction, cryptorrhea, and infectious lesions may become pathogenic factors. Occasionally, antidepressants are used clinically to treat intractable pruritus, neurodermatitis and other skin conditions, such as trimipramine and chlorpromazine, which are helpful in the treatment of pruritus and sleep disturbances that are common in chronic skin diseases. However, these antidepressants have many side effects and may also interact with drugs for skin, and not every antidepressant can be used for the treatment of skin disease such as neurodermatitis.

Cannabis (scientific name: Cannabis sativa L.), a plant of cannabis family, cannabis genus, also known as hemp, Chinese hemp, Huo hemp, Shansi Miao, and jute, has important agricultural and medicinal values. Cannabis contains a toxic component THC (tetrahydrocannabinol) that can cause hallucinations and addiction, and thus can be used as a drug. Cannabis has been banned to be planted for quite a long time. Due to the high economic and medicinal value of cannabis, raw material cannabis is applied exclusively for industrial use and referred to as "industrial hemp", which contains less than three thousandths of tetrahydrocannabinol (THC) in cannabis flowers and leaves during the growing period. The industrial hemp does not have the value of extracting the toxic component tetrahydrocannabinol or directly taken as a drug, and can be legally cultivated on a large scale and used for industrial development.

Cannabinoid is an active substance extracted or synthesized from a natural plant, cannabis. Currently, more than 500 substances have been isolated from the cannabis plant, wherein there are at least 86 cannabinoid compounds. Cannabinoid compounds are a class of special substances in the cannabis plant, and the main active ingredients in the cannabis plant. Researches on the cannabinoid compounds have always been a hot spot in cannabis research. The main cannabinoid compounds in the cannabis plant include tetrahydrocannabinol (THC), cannabinol (CBN), cannabidiol (CBD), cannabidivarin (CBDV), tetrahydrocannabinovarin (THCV), etc., and the first three of which account for 90% or above of the cannabinoid compounds.

TV Zanelati et al. (Antidepressant-like effects of cannabidiol in mice: possible involvement of 5-HT1A receptors, British journal of pharmacology, 2010) found that cannabidiol may induce antidepressant-like effects through activation of 5-HT_{1A} receptors and believed that cannabidiol has antidepressant activity in mice. Patent US2014302086 discloses a small molecule composition of THC or cannabidiol and at least one selected from citric acid, ascorbic acid, citrus essential oil, etc., and mentions that the above composition may treat neurodermatitis. Patent WO0206999A2 discloses a medication containing at least 80 wt% of cannabinoid, wherein the weight ratio of THC to CBD is 75:25-20:80, and mentions that the above composition may be used for the treatment of neurodermatitis. However, the above prior arts only briefly list the therapeutic uses of said compositions, and the effect of THC or cannabidiol in neurodermatitis was not specifically verified in the specifications. Furthermore, the composition in US2014302086 also contains other ingredients capable of relieving neurodermatitis, and it is difficult to clarify the effect of THC or cannabidiol. That is to say, the conclusion that CBD or THC can treat neurodermatitis cannot be clearly drawn from the above prior arts.

The inventors of the present application have conducted extensive researches to verify the effect of cannabinoid compounds derived from the natural plant in the treatment of neurodermatitis. The inventors screened among various cannabinoid compounds and performed numerous clinical trials, and finally succeeded in determining several cannabinoid compounds and the combinations thereof which have significant efficacy in neurodermatitis.

### SUMMARY

After reading the detailed description of the preferred embodiments and the appended claims, the objects, advantages and uses of the present invention will be revealed to those skilled in the art. The present invention is intended to address the existing deficiencies in the treatment of neurodermatitis and finds that the cannabinoid compounds can treat neurodermatitis and can be used in the preparation of medications for treating neurodermatitis.

The present invention provides a method of treating neurodermatitis by using these compositions, and use of said compositions in preparation of a medicament for treating neurodermatitis.

The invention is defined in claims 1 to 4.

The present invention provides a pharmaceutical composition comprising a cannabinoid combination or pharmaceutically available salts thereof for use in the treatment of neurodermatitis, , wherein the cannabinoid combination is :
the combination of cannabidiol (CBD), cannabidivarin (CBDV) and tetrahydrocannabinovarin (THCV) has a ratio of cannabidiol (CBD) to cannabidivarin (CBDV) to tetrahydrocannabinovarin (THCV) of 100:20-50: 2.5-10 by weight.

The pharmaceutical composition further comprises one or more pharmaceutically acceptable carriers or excipients.

The pharmaceutically available salts described in the invention include acid addition salts formed with inorganic or organic acids, said inorganic acids such as hydrochloric acid, hydrobromic acid, sulfuric acid, nitric acid, phosphoric acid, etc.; said organic acids such as acetic acid, propionic acid, hexanoic acid, heptanoic acid, pyruvic acid, lactic acid, malonic acid, butanedioic acid, malic acid, maleic acid, fumaric acid, tartaric acid, citric acid, benzoic acid, o-(4-hydroxybenzoyl)benzoic acid, cinnamic acid mandelic acid, methanesulfonic acid, ethanesulfonic acid, tert-valeric acid, tert-butylacetic acid, dodecylsulfuric acid, gluconic acid, glutamic acid, naphthoic acid, salicylic acid, stearic acid, etc.

The composition described in the invention can be prepared into specific dosage forms for administration by any suitable route such as oral, rectal, nasal, pulmonary, topical (including buccal and sublingual), transdermal, intracisternal, intraperitoneal, vaginal and parenteral (including subcutaneous, intramuscular, intrathecal, intravenous and intradermal) routes, preferably the oral route. It should be understood that the preferred route depends on the general condition and age of the patient to be treated, the nature of the disease to be treated and the specific active ingredient or selected active ingredient.

The composition for oral administration includes solid dosage forms, such as capsules, tablets, sugar-coated tablets, pills, lozenges, powders and granules.

The composition for oral administration further includes liquid dosage forms, such as solutions, emulsions, suspensions, syrups, and elixirs.

The composition for parenteral administration includes sterile aqueous and non-aqueous injectable solutions, dispersions, suspensions or emulsions, and sterile powders that are redissolved in sterile injectable solutions or dispersions prior to use.

Other suitable dosage forms for administration include suppositories, sprays, creams, gels, inhalers, patches, and implants, and so on.

The composition of the present invention or the composition prepared according to the present invention may be administrated by any suitable route, for example, by oral administration in the form of tablets, capsules, powders, syrups, and so on, or by parenteral injection in the form of solutions.

To prepare such composition, methods known in the art may be used and any pharmaceutically acceptable carrier, diluent, excipient or other additive commonly used in the art may be employed.

For parenteral administration, sterile aqueous solutions, aqueous propylene glycol solutions, aqueous vitamin E solutions, or sesame oil or peanut oil solutions of one or more active ingredients may be used. If necessary, such aqueous solutions should be properly buffered and a liquid diluent may be first prepared to be isotonic by using sufficient salt or glucose. The aqueous solutions are particularly suitable for intravenous, intramuscular, subcutaneous and intraperitoneal administration. The employed sterile aqueous media may be readily made by standard techniques known to those skilled in the art.

The solutions for injection may be prepared by dissolving one or more active ingredients and possible additives into a portion of a solvent for injection (preferably sterile water), adjusting the solution to the desired volume, sterilizing the solution and pouring the sterilized solution into a suitable ampoule or vial. Any appropriate additives commonly used in the art, such as tension agents, preservatives, and antioxidants, may be added.

Suitable drug carriers include inert solid diluents or fillers, sterile aqueous solutions, and various organic solvents.

Examples of solid carriers include lactose, white clay, sucrose, cyclodextrin, talc, agar, pectin, arabic gum, stearic acid, lower alkyl ethers of cellulose, corn starch, potato starch, talc, magnesium stearate, gelatin, and so on.

Any other adjuvants or additives normally used for coloring, flavoring, preserving or the like may be used as long as they are compatible with the active ingredient or ingredients already used.

Examples of liquid carriers include molasses, peanut oil, olive oil, phospholipid, fatty acid, fatty acid amine, polyoxyethylene and water. Similarly, the carrier or diluent may include any slow-release material known in the art, such as glycerol monostearate or glycerol distearate, alone or mixed with wax.

The composition formed by mixing one or more active ingredients of the present invention with a pharmaceutically acceptable carrier may be conveniently administered in a variety of dosage forms suitable for the disclosed route of administration. The preparation may be present conveniently in a unit dosage form by methods known in the field of pharmacology.

The active ingredients of the present invention can be formulated into similar or dissimilar pharmaceutical compositions and unit dosage forms thereof.

Preferably, the pharmaceutical composition of the present invention contains a unit dose of 2.5-400 mg of a cannabinoid compound or a pharmaceutically available salt thereof, more preferably, the pharmaceutical composition contains a unit dose of 25-300 mg of a cannabinoid compound or a pharmaceutically available salt thereof, most preferably, the pharmaceutical composition contains a unit dose of 50-200 mg of a cannabinoid compound or a pharmaceutically available salt thereof. In embodiments of the present invention, the pharmaceutical composition contains a unit dose of 25mg, 30mg, 40mg, 50mg, 60mg, 70mg, 80mg, 90mg, 100mg, 110mg, 120mg, 130mg, 140mg, 150mg, 160mg, 170mg, 180mg, 190mg or 200mg of a cannabinoid compound or a pharmaceutically available salt thereof.

The cannabinoid compound or the pharmaceutically available salt thereof of the present invention may be a chemically synthesized product, a biosynthesized product, a plant extract or a product prepared by other means. Preferably, the cannabinoid compound of the present invention is the plant extract, and the plant may be the stalk cores, flowers, leaves, roots and/or outer shells of seeds of *Cannabis sativa L.*

In the case that the cannabinoid compound of the present invention is the plant extract, the extraction solvent may be a low molecular alcohol (such as methanol, ethanol, butanol or propanol); acetate (such as methyl acetate or ethyl acetate); ketone (such as acetone); ether (such as methyl ether or diethyl ether); low-boiling-point aliphatic or aromatic hydrocarbon or chlorinated hydrocarbon. The method for extraction includes:
(1) the stalk cores, flowers, leaves, roots and/or outer shells of seeds of *Cannabis sativa L.* are heated to reflux by using about 3-10 times the weight of the above extraction solvent or a mixture thereof, preferably for at least about 1 hour, followed by filtration to remove the residue, and then the solvent is removed, preferably the solvent is removed under vacuum. The resultant extractum is heated at a temperature of about 110-135°C for about 40 minutes, followed by chromatographic separation, preferably with a mobile phase mixture for chromatography consisting of methanol/water and acetic acid or ethanol/water and acetic acid.
(2) the stalk cores, flowers, leaves, roots and/or outer shells of seeds of *Cannabis sativa L.* are heated to reflux by using about 3-10 times the weight of the above extraction solvent or a mixture thereof, preferably for at least about 1 hour, followed by filtration, and then extraction is conducted at least twice with a 1-10% aqueous sodium hydroxide solution that preferably contains about 20 wt% of ethanol. The extract is mixed with a 5% sulfuric acid solution to give a pH of about 2-4, then, the mixture is subjected to extraction at least twice with a low-boiling-point solvent (e.g., low-boiling-point aliphatic hydrocarbon, aromatic hydrocarbon, chlorinated hydrocarbon, methyl acetate, ethyl acetate, or a mixture thereof), and then the solvent is removed at low temperature under vacuum, followed by chromatographic separation, preferably with a mobile phase mixture for chromatography consisting of methanol/water and acetic acid or ethanol/water and acetic acid.

The patient of the present invention is a mammal, such as humans, mice, rats, guinea pigs, dogs, cats, horses, cows, pigs, or non-human primates such as monkeys, chimpanzees or baboons. Preferably, the patient is a human.

Unless otherwise specified, the term "pharmaceutically acceptable carrier or excipient" means that the ingredient is free of biologically active or other undesirably active impurities, and the ingredient, for example, may be incorporated into the disclosed pharmaceutical formulation and administered to a patient without causing significant adverse biological effects or interacting with other ingredients contained in the preparation in a deleterious manner.

Unless otherwise specified, the term "treating" includes inhibiting, delaying, alleviating, attenuating, limiting, relieving or eliminating a disease, disorder, condition or state, or occurrence and/or progression thereof, and/or symptoms thereof.

Unless otherwise specified, the term "including" denotes "open" or "inclusive" terms such that the term includes the listed elements and further includes additional, unmentioned elements.

Unless otherwise stated, the term "about" usually means +/-5% of the stated value, more usually +/-4% of the stated value, more usually +/-3% of the stated value, more usually +/-2% of the stated value, more usually +/-1% of the stated value, more usually +/-0.5% of the stated value.

The present invention has verified through numerous studies that the cannabinoid compound or the pharmaceutically available salts thereof are significantly effective in the treatment of neurodermatitis, and that the cannabinoid compound alone, as well as the combinations of the above, may have the ability to improve various symptoms of neurodermatitis and can be used in the preparation of drugs for the treatment of neurodermatitis.

### DETAILED DESCRIPTION

It is to be noted that the embodiments and the features in the embodiments of the present application may be combined with each other without conflict. The present invention will be described in detail below in connection with the embodiments.

### Embodiment 1. Capsules in a unit dosage form (not part of the present invention)

Prescription ingredients:
Cannabidiol 200mg
Microcrystalline cellulose 90mg
Pregelatinized starch 100mg
Cross-linked carboxymethyl cellulose 7mg
Magnesium stearate 0.5mg

Preparation method: the active ingredient was sieved and mixed with the excipients, and the mixture was packed into hard gelatin capsules.

### Embodiment 2. Capsules in a unit dosage form (not part of the present invention)

Prescription ingredients:
Cannabidiol 400mg
Microcrystalline cellulose 100mg
Pregelatinized starch 150mg
Cross-linked carboxymethyl cellulose 10mg
Magnesium stearate 0.2mg

Preparation method was the same as that in Embodiment 1.

### Embodiment 3. Tablets in a unit dosage form (not part of the present invention)

Prescription ingredients:
Tetrahydrocannabinol 100mg
Dextrin 100mg
Microcrystalline cellulose 25mg
Polyvinylpyrrolidone 10mg
Sodium carboxymethyl starch 15mg
Magnesium stearate 1mg

Preparation method: the active ingredient was sieved and mixed with dextrin, microcrystalline cellulose, polyvinylpyrrolidone and sodium carboxymethyl starch until a homogeneous mixture was formed. The homogeneous mixture was sieved and mixed with magnesium stearate. The resulting powder mixture was then pressed into tablets of the desired shape and size.

### Embodiment 4. Tablets in a unit dosage form (not part of the present invention)

Prescription ingredients:
Tetrahydrocannabinol 40mg
Pregelatinized starch 150mg
Microcrystalline cellulose 25mg
Sodium carboxymethyl starch 15mg
Magnesium stearate 1mg

Preparation method: the active ingredient was sieved and mixed with pregelatinized starch, microcrystalline cellulose and sodium carboxymethyl starch until a homogeneous mixture was formed. The homogeneous mixture was sieved and mixed with magnesium stearate. The resulting powder mixture was then pressed into tablets of the desired shape and size.

### Embodiment 5. Tablets in a unit dosage form (not part of the present invention)

Prescription ingredients:
Cannabidiol 200mg
Cannabidivarin 40mg
Dextrin 150mg
Sodium carboxymethyl starch 15mg
Magnesium stearate 1mg

Preparation method: the active ingredients were sieved and mixed with dextrin and sodium carboxymethyl starch until a homogeneous mixture was formed. The homogeneous mixture was sieved and mixed with magnesium stearate. The resulting powder mixture was then pressed into tablets of the desired shape and size.

### Embodiment 6. Tablets in a unit dosage form (not part of the present invention)

Prescription ingredients:
Tetrahydrocannabinol 100mg
Cannabidivarin 40mg
Pregelatinized starch 150mg
Microcrystalline cellulose 25mg
Sodium carboxymethyl starch 15mg
Magnesium stearate 1mg

Preparation method: the active ingredients were sieved and mixed with pregelatinized starch, microcrystalline cellulose and sodium carboxymethyl starch until a homogeneous mixture was formed. The homogeneous mixture was sieved and mixed with magnesium stearate. The resulting powder mixture was then pressed into tablets of the desired shape and size.

### Embodiment 7. Tablets in a unit dosage form (not part of the present invention)

Prescription ingredients:
Cannabidiol 200mg
Tetrahydrocannabinovarin 10mg
Pregelatinized starch 150mg
Microcrystalline cellulose 25mg
Polyvinylpyrrolidone 10mg
Sodium carboxymethyl starch 15mg
Magnesium stearate 1mg

Preparation method: the active ingredients were sieved and mixed with pregelatinized starch, microcrystalline cellulose, polyvinylpyrrolidone and sodium carboxymethyl starch until a homogeneous mixture was formed. The homogeneous mixture was sieved and mixed with magnesium stearate. The resulting powder mixture was then pressed into tablets of the desired shape and size.

### Embodiment 8. Tablets in a unit dosage form (not part of the present invention)

Prescription ingredients:
Tetrahydrocannabinol 100mg
Tetrahydrocannabinovarin 10mg
Pregelatinized starch 150mg
Microcrystalline cellulose 25mg
Polyvinylpyrrolidone 10mg
Sodium carboxymethyl starch 15mg
Magnesium stearate 1mg

Preparation method: the active ingredients were sieved and mixed with pregelatinized starch, microcrystalline cellulose, polyvinylpyrrolidone and sodium carboxymethyl starch until a homogeneous mixture was formed. The homogeneous mixture was sieved and mixed with magnesium stearate. The resulting powder mixture was then pressed into tablets of the desired shape and size.

### Embodiment 9. Tablets in a unit dosage form (not part of the present invention)

Prescription ingredients:
Tetrahydrocannabinol 100mg
Cannabidivarin 40mg
Tetrahydrocannabinovarin 10mg
Dextrin 150mg
Sodium carboxymethyl starch 15mg
Magnesium stearate 1mg

Preparation: the active ingredients were sieved and mixed with dextrin and sodium carboxymethyl starch until a homogeneous mixture was formed. The homogeneous mixture was sieved and mixed with magnesium stearate. The resulting powder mixture was then pressed into tablets of the desired shape and size.

### Embodiment 10. Tablets in a unit dosage form

Prescription ingredients:
Cannabidiol 200mg
Cannabidivarin 40mg
Tetrahydrocannabinovarin 10mg
Dextrin 150mg
Sodium carboxymethyl starch 15mg
Magnesium stearate 1mg

Preparation method: the active ingredients were sieved and mixed with dextrin and sodium carboxymethyl starch until a homogeneous mixture was formed. The homogeneous mixture was sieved and mixed with magnesium stearate. The resulting powder mixture was then pressed into tablets of the desired shape and size.

### Embodiment 11. Injection in unit dosage form (10mL of aqueous injection) (not part of the present invention)

Prescription ingredients:
Tetrahydrocannabinovarin 500mg
Hydroxypropyl-β-cyclodextrin q.s.
0.1 mol/LHCl q.s.
Sodium chloride q.s.
Water for injection to 1000mL

Preparation method: about 800ml of the water for injection was taken, and hydroxypropyl-β-cyclodextrin (q.s.) was added, followed by addition of tetrahydrocannabinovarin component to be dissolved, then the pH was adjusted to 6.2-6.5 with 0.1mol/L HCl, the water for injection was added to the full amount, uniform stirring was conducted, then sodium chloride was added to blend to be isotonic, filtering was conducted, and the filtered material was sealed into an ampoule, and sterilized by steam at 121°C for 15min. 10ml of the sterilized material was filled.

### Embodiment 12. Injection in unit dosage form (10mL of powder injection) (not part of the present invention)

Prescription ingredients:
Cannabidivarin 4000mg
Mannitol 50g
0.1mol/L HCl q.s.
Hydroxypropyl-β-cyclodextrin q.s.
Sodium chloride q.s.
Water for injection to 1000mL

Preparation method: about 800ml of the water for injection was taken, mannitol in a prescribed dose and hydroxypropyl-β-cyclodextrin (q.s.) were added, then respective cannabinoid active ingredient was added to be dissolved, the pH was adjusted to 6.2-6.5 with stirring by adding an appropriate amount of 0.1 mol/L HCl, then the water for injection was added to the full amount, sodium chloride was added to blend to be isotonic, filtering was conducted, and the filtered material was freeze-dried according to the process of lyophilized powder injection to prepare the powder injection. 10ml of the powder injection was filled.

### Embodiment 13. Impact of administration of cannabinoid compound alone on the itch-causing effect of histamine phosphate (not part of the present invention)

### Experimental principle:

Itching sensation is modulated by the central nervous system, and the modulation is achieved through the excitatory and inhibitory circuits of neurons located in the spinal cord and the opioid-like system. Therefore, psychological factors can cause neuroimmune changes that dysregulate the excitatory and inhibitory functions of the central nervous system, resulting in the disorder of the modulation mechanism of itching sensation to induce central itching sensation. Cannabinoid compounds are able to modulate the central nervous system through cannabinoid CB 1 and/or CB2 receptors to achieve alleviation of itching.

### Implementation method:

70 guinea pigs were taken and randomly and equally divided into a blank control group (100mg/day of distilled water), a cannabidiol group (50mg/day, 100mg/day, 200mg/day), a tetrahydrocannabinol group (40mg/day, 80mg/day, 100mg/day), a cannabidivarin group (40mg/day, 80mg/day, 100mg/day), a tetrahydrocannabinovarin group (5mg/day, 10mg/day, 20mg/day), and a control group (10mg/day of chlorphenamine maleate), 5 guinea pigs in each group. The guinea pigs in each group were shaved 2cm×2cm on the dorsal surface of the right hind foot and administered orally for 3 consecutive days according to the above administration dosage, respectively.

On day 3 of administration, the shaved area was abraded with coarse sandpaper to the extent that the epidermis was injured with mild bleeding. After 10 minutes, 0.05 ml of 0.01% histamine phosphate solution was added dropwise to the wounded area of each guinea pig, respectively, after that, the histamine phosphate solutions with increased concentration of histamine phosphate (e.g., 0.02%/0.03%/0.04%/0.05%/0.06%, etc.) were sequentially added dropwise every other 3 min until the guinea pigs turned back and licked their feet. The total amount of histamine given to each guinea pig was recorded, which was the itch-causing threshold.

### Experimental results:

| group | administration dosage | itch-causing threshold /µL |
|---|---|---|
| blank control group | 100mg/day of distilled water | 44.35±38.12 |
| cannabidiol group | 50mg/day | 90.24±45.98 |
| | 200mg/day | 96.55±42.34 |
| | 400mg/day | 117.67±44.67 |
| tetrahydrocannabinol group | 40mg/day | 90.65±32.08 |
| | 80mg/day | 92.10±50.78 |
| | 100mg/day | 94.98±48.96 |
| cannabidivarin group | 40mg/day | 56.23±45.87 |
| | 80mg/day | 59.55±39.84 |
| | 100mg/day | 56.89±34.25 |
| tetrahydrocannabinovarin group | 5mg/day | 50.45±45.67 |
| | 10mg/day | 56.43±34.18 |
| | 20mg/day | 55.23±40.92 |
| control group | 10mg/day of | 127.03±54.05 |
| | chlorphenamine maleate | |

It can be seen from this experiment that the administration of cannabidiol and tetrahydrocannabinol alone significantly alleviates the itch-causing effect of histamine phosphate.

### Embodiment 14. Impact of administration of composition of cannabinoid compound on the itch-causing effect of histamine phosphate

Although the administration of cannabidiol and tetrahydrocannabinol alone showed a certain relief of the itch-causing effect of histamine phosphate, it would be more desirable to be able to find active ingredients with better effects. Therefore, this experiment compared the impact of different groups of the compositions of cannabinoid compounds on the itch-causing effect of histamine phosphate.

### Experimental method:

90 guinea pigs were taken and randomly and equally divided into:
a tetrahydrocannabinol (THC) and tetrahydrocannabinovarin (THCV) combination group;
a cannabidiol (CBD) and cannabidivarin (CBDV) combination group;
a tetrahydrocannabinol (THC) and cannabidivarin (CBDV) combination group;
a cannabidiol (CBD) and tetrahydrocannabinovarin (THCV) combination group;
a cannabidiol (CBD), cannabidivarin (CBDV) and tetrahydrocannabinovarin (THCV) combination group;
a tetrahydrocannabinol (THC), cannabidivarin (CBDV) and tetrahydrocannabinovarin (THCV) combination group:
   5 guinea pigs in each group, experimental steps the same as that in Embodiment 13.

### Experimental results:

| group | administration dosage | itch-causing threshold /µL |
|---|---|---|
| THC+THCV combination group | 100mg/day of THC+5mg/day of THCV | 98.31±33.23 |
| | 100mg/day of THC+10mg/day of THCV | 109.78±50.34 |
| | 100mg/day of THC+20mg/day of THCV | 112.01±52.18 |
| CBD+CBDV combination group | 200mg/day of CBD+40mg/day of CBDV | 103.34±44.14 |
| | 200mg/day of CBD+80mg/day of CBDV | 122.10±56.32 |
| | 200mg/day of CBD+100mg/day of CBDV | 128.98±45.68 |
| THC+CBDV combination group | 100mg/day of THC+40mg/day of CBDV | 102.11±39.78 |
| | 100mg/day of THC+ 80mg/day of CBDV | 104.68±44.47 |
| | 100mg/day of THC+100mg/day of CBDV | 111.76±50.98 |
| CBD+THCV combination group | 200mg/day of CBD+5mg/day of THCV | 101.45±46.98 |
| | 200mg/day of CBD+10mg/day of THCV | 111.45±45.87 |
| | 200mg/day of CBD+20mg/day of THCV | 119.76±25.98 |
| CBD+CBDV+THCV combination group | 200mg/day of CBD+40mg/day of CBDV+5mg/day of THCV | 131.87±46.87 |
| | 200mg/day of CBD+80mg/day of CBDV+10mg/day of THCV | 139.13±54.09 |
| | 200mg/day of CBD+100mg/day of CBDV+20mg/day of THCV | 140.56±42.34 |
| THC+CBDV+THCV combination group | 100mg/day of THC+40mg/day of CBDV+5mg/day of THCV | 112.21±54.97 |
| | 100mg/day of THC+80mg/day of CBDV+10mg/day of THCV | 118.56±35.87 |
| | 100mg/day of THC+100mg/day of CBDV+20mg/day of THCV | 124.77±35.35 |

From this experiment, it can be seen that the combination of two or more cannabinoid compounds has a superior effect compared with the administration of cannabidiol as well as tetrahydrocannabinol alone, which may be related to the fact that different cannabinoid compounds act on different receptor targets.

### Embodiment 15. Clinical study of cannabinoid compound for neurodermatitis

Patients with generalized neurodermatitis in dermatology outpatient and inpatient departments were selected.

Diagnostic criteria: all meet the diagnosis of neurodermatitis in Clinical Dermatology. Its clinical manifestations are mainly characterized by skin lichen-like changes and intense itching. It mostly occurs on the back of the neck or both sides thereof, cubital fossae, popliteal fossae, forearms, thighs, calves and a lumbosacral area. Subjective symptoms are often severe paroxysmal itching, even at night. Most patients have dizziness, insomnia, irritability, anxiety and other symptoms of neurosism.

Exclusion criteria: (1) patients with skin lesions with bacterial or fungal infections; (2) pregnant or lactating women; (3) patients with local administration of glucocorticoid drugs or antihistamines within 1 week before selection; (4) patients with systemic administration of glucocorticoids within 1 month; (5) patients who are known to be allergic to the drug for study and its matrix components; (6) patients with chronic liver or kidney disease or other serious illness; (7) patients with diabetes mellitus or severe immunocompromise.

90 patients with diagnosed neurodermatitis were selected for clinical observation, and the patients were randomly divided into nine groups, including eight cannabinoid compound treatment groups, a total of 80 people, 56 men and 24 women, aged 30 to 61 years, using preparations prepared in Embodiments 1/3/5/6/7/8/9/10, with 10 cases sex randomly selected in each group. 10 people was in a doxepin hydrochloride group as a control group, 5 males and 5 females, aged 30 to 61 years.

Treatment method: the cannabinoid compound treatment groups took the preparations prepared in Embodiments 1/3/5/6/7/8/9/10 orally, once per day, at bedtime, for 2 consecutive weeks; the control group used 25 mg/d of doxepin hydrochloride, orally, once per day, at bedtime, for 2 consecutive weeks.

The observation indexes included pruritus degree, inflammation degree, scales hypertrophy degree and target skin lesion surface, and the most serious skin lesion site was selected as the target skin lesion site, and the area of the target skin lesion, clinical symptoms and changes in physical signs were recorded at each follow-up visit without any other drugs during the treatment period. Local observation and evaluation of the skin lesions were performed weekly in 1 week before treatment, 2 weeks during treatment and 1 month after discontinuation of the drug. Skin lesions were scored as follows:

| observation item | scores | | | | |
|---|---|---|---|---|---|
| | 0 | 1 | 2 | 3 | 4 |
| Pruritus degree | No | extremely mild, mildly aware, easily tolerated, without scratching | mild, aware, disturbed but tolerated, sometimes scratching | moderate, clearly aware, interferes with daily activities and sleep, but able to get enough sleep | frequent scratching strongly, obviously aware, seriously affects daily life and sleep, poor sleep, waking up 1-2 times |
| inflammation degree | No | slightly red | slightly infiltrated more red | infiltrated flushed | infiltrated obviously |
| scales | | | scales, with skin | significant scales and | |
| hypertrophy | No | mildly scales, no lichenification | lesions of mild | skin lesions of thicker | severe lichenification |
| degree | | | lichenification | lichenification | |
| target skin | completely | 75%-100% | 50%-74% | 25%-49% reduction in | 1%-24% or no |
| lesion area | faded | reduction in area | reduction in area | area | reduction in area |

Criteria for determining efficacy: the scoring criteria were that each index was scored according to a 4-level scoring method in each evaluation, i.e., 0 for none, 1 for mild, 2 for moderate, and 3 for severe. All patients were required to have an erosion score of <1 at the time of enrollment, and the skin lesion area was always recorded as 3, and the total value of each index score was recorded. Efficacy judgment: the efficacy was judged by the percentage reduction of the score value as the efficacy index, and the formula for calculating the efficacy index was: (total score at the initial consultation - total score at each follow-up visit) / total score at the initial consultation × 100%. An efficacy index of >90% was considered cured, an efficacy index of 61-89% was considered excellently effective, an efficacy index of 20-60% was considered effective, and an efficacy index of <20% was considered ineffective.

The results of the clinical trials were as follows:

| group | n | Cu red | excell ently effecti ve | effe ctiv e | inef feet ive |
|---|---|---|---|---|---|
| 200mg of cannabidiol | 10 | 1 | 3 | 6 | 0 |
| 100mg of tetrahydrocannabinol | 10 | 1 | 2 | 6 | 1 |
| 200mg of cannabidiol+40mg of cannabidivarin | 10 | 3 | 5 | 2 | 0 |
| 100mg of tetrahydrocannabinol+40mg of cannabidivarin | 10 | 0 | 6 | 3 | 1 |
| 100mg of tetrahydrocannabinol+10mg of tetrahydrocannabinovarin | 10 | 1 | 4 | 4 | 1 |
| 200mg of cannabidiol+10mg of tetrahydrocannabinovarin | 10 | 2 | 4 | 3 | 1 |
| 200mg of cannabidiol+40mg of cannabidivarin +10mg of tetrahydrocannabinovarin group | 10 | 4 | 3 | 3 | 0 |
| 100mg of tetrahydrocannabinol+40mg of cannabidivarin | 10 | 2 | 3 | 4 | 1 |

| | | | | | |
|---|---|---|---|---|---|
| +10mg of tetrahydrocannabinovarin group | | | | | |
| doxepin hydrochloride control group | 10 | 0 | 6 | 3 | 1 |

From this experiment, it can be seen that the administration of cannabidiol and tetrahydrocannabinol alone and the combination of two or more cannabinoid compounds have clinical efficacy in neurodermatitis, and the combination of two or more cannabinoid compounds administrated at same time has a superior effect.

## Claims

1. A pharmaceutical composition comprising a cannabinoid combination or pharmaceutically available salts thereof for use in the treatment of neurodermatitis, wherein the cannabinoid combination is :
the combination of cannabidiol (CBD), cannabidivarin (CBDV) and
tetrahydrocannabinovarin (THCV) has a ratio of cannabidiol (CBD) to cannabidivarin (CBDV) to tetrahydrocannabinovarin (THCV) of 100:20-50: 2.5-10 by weight.

2. The pharmaceutical composition for use according to claim 1 further comprising one or more pharmaceutically acceptable carriers or excipients.

3. The composition for use according to any one of claims 1-2, **characterized in that** the pharmaceutical composition is selected from the group consisting of capsules, tablets, pills, lozenges, granules, solutions, emulsions, suspensions, syrups, sterile injections, sterile powders, suppositories, sprays, ointments, creams, gels, inhalants, dermal patches or implants.

4. A method for preparing the composition for use of any one of claims 1-2 comprising the step of: uniformly mixing the cannabinoid compounds, or pharmaceutically available salts thereof in proportion to obtain the composition.

## Patentansprüche

1. Eine pharmazeutische Zusammensetzung, die eine Cannabinoid-Kombination oder pharmazeutisch verfügbare Salze davon beinhaltet, zur Verwendung bei der Behandlung von Neurodermatitis, wobei die Cannabinoid-Kombination Folgendes ist:
die Kombination aus Cannabidiol (CBD), Cannabidivarin (CBDV) und
Tetrahydrocannabinovarin (THCV) weist ein Gewichtsverhältnis von Cannabidiol (CBD) zu Cannabidivarin (CBDV) zu Tetrahydrocannabinovarin (THCV) von 100:20-50:2,5-10 auf.

2. Pharmazeutische Zusammensetzung zur Verwendung gemäß Anspruch 1, die ferner einen oder mehrere pharmazeutisch akzeptable Träger oder Hilfsstoffe beinhaltet.

3. Zusammensetzung zur Verwendung gemäß einem der Ansprüche 1-2, **dadurch gekennzeichnet, dass** die pharmazeutische Zusammensetzung ausgewählt ist aus der Gruppe, bestehend aus Kapseln, Tabletten, Pillen, Lutschtabletten, Granulaten, Lösungen, Emulsionen, Suspensionen, Sirupen, sterilen Injektionen, sterilen Pulvern, Suppositorien, Sprays, Salben, Cremes, Gelen, Inhalationsmitteln, Hautpflastern oder Implantaten.

4. Ein Verfahren zum Vorbereiten der Zusammensetzung zur Verwendung gemäß einem der Ansprüche 1-2, das den folgenden Schritt beinhaltet: einheitliches Mischen der Cannabinoid-Verbindungen oder pharmazeutisch verfügbarer Salze davon in proportionaler Weise, um die Zusammensetzung zu erhalten.

## Revendications

1. Une composition pharmaceutique comprenant une combinaison de cannabinoïde ou des sels pharmaceutiquement disponibles de celle-ci, pour utilisation dans le traitement de la neurodermatite, dans laquelle la combinaison de cannabinoïde est :
la combinaison de cannabidiol (CBD), de cannabidivarine (CBDV) et de tétrahydrocannabinovarine (THCV) a un rapport du cannabidiol (CBD) à la cannabidivarine (CBDV) à la tétrahydrocannabinovarine (THCV) de 100/20-50/2,5-10 en poids.

2. La composition pharmaceutique pour utilisation selon la revendication 1 comprenant de surcroît un ou plusieurs véhicules ou excipients pharmaceutiquement acceptables.

3. La composition pour utilisation selon l'une quelconque des revendications 1 à 2, **caractérisée en ce que** la composition pharmaceutique est sélectionnée dans le groupe constitué de gélules, comprimés, pilules, pastilles, granulés, solutions, émulsions, suspensions, sirops, injections stériles, poudres stériles, suppositoires, sprays, pommades, crèmes, gels, inhalants, patchs dermiques ou implants.

4. Un procédé pour préparer la composition pour utilisation de l'une quelconque des revendications 1 à 2 comprenant l'étape de : mélanger uniformément les composés de cannabinoïde, ou sels de ceux-ci disponibles d'un point de vue pharmaceutique, en proportion afin d'obtenir la composition.
